# EUROPÄISCHE PATENTSCHRIFT

(11) **EP 1 059 911 B1**
(45) Veröffentlichungstag und Bekanntmachung des Hinweises auf die Patenterteilung: **20.04.2005**
(21) Anmeldenummer: 99913176.6
(22) Anmeldetag: 20.02.1999
(51) Int. Cl.: A61K 7/42, A61K 31/35

(54) **FORMULIERUNGEN MIT ANTIVIRALER WIRKUNG**
FORMULATIONS WITH AN ANTI-VIRAL EFFECT
FORMULATIONS A ACTION ANTIVIRALE

(30) Priorität: 05.03.1998 DE 19809304
(43) Veröffentlichungstag der Anmeldung: 20.12.2000
(73) Patentinhaber: MERCK PATENT GmbH, 64293 Darmstadt (DE)
(72) Erfinder: BUCHHOLZ, Herwig, D-60599 Frankfurt (DE); KRAUS, Christine, D-67165 Waldsee (DE); WAGNER, Annette, D-60435 Frankfurt (DE); MEDUSKI, Jerzy, Playa Del Rey, CA 90293-7640 (US)
(86) Internationale Anmeldenummer: PCT/EP1999/001104
(87) Internationale Veröffentlichungsnummer: WO 1999/044578

(56) Entgegenhaltungen:
- DE-A- 19 508 608
- FR-A- 2 723 316
- US-A- 4 132 782
- CHEMICAL ABSTRACTS, vol. 120, no. 7, 14. Februar 1994 (1994-02-14) Columbus, Ohio, US; abstract no. 69601, XP002110525 & JP 05 271088 A (RUIBOSUTEI) 19. Oktober 1993 (1993-10-19)
- CHEMICAL ABSTRACTS, vol. 120, no. 7, 14. Februar 1994 (1994-02-14) Columbus, Ohio, US; abstract no. 69623, XP002110526 & JP 05 271090 A (RUIBOSUTEI) 19. Oktober 1993 (1993-10-19)
- CHEMICAL ABSTRACTS, vol. 121, no. 21, 21. November 1994 (1994-11-21) Columbus, Ohio, US; abstract no. 246288, XP002110527 & JP 06 199697 A (YUNIE KK) 19. Juli 1994 (1994-07-19)
- DATABASE WPI Week 8548 Derwent Publications Ltd., London, GB; AN 85300541 XP002110528 & JP 60 208908 A (KANEBO), 21. Oktober 1985 (1985-10-21)
- DATABASE WPI Week 199219, Derwent Publications Ltd., London, GB; AN 1992-157315 & JP 4 099 730 A (SAN-EI CHEM IND LTD) 31 M{rz 1992

## Beschreibung

Die vorliegende Erfindung betrifft Formulierungen in fester oder flüssiger Form, enthaltend Isoquercetin als natürliches Flavonoid, welches darin als Lichtschutzfilter und antiviral wirkende Substanz enthalten ist. Die Erfindung betrifft sowohl kosmetische als auch medizinische Formulierungen.

Bekanntlich reagiert die Haut empfindlich auf Sonnenstrahlen, welche einen gewöhnlichen Sonnenbrand oder ein Erythem, aber auch mehr oder weniger ausgeprägte Verbrennungen hervorrufen können.

Sonnenstrahlen haben aber auch andere negative Wirkungen: sie be-wirken, daß die Haut ihre Elastizität verliert und sich Falten bilden und führen somit zu einer frühzeitigen Alterung. Manchmal kann man auch Dermatosen beobachten. Im extremen Fall kommt es bei manchen Menschen zum Auftreten von Hautkrebs. Weiterhin ist bekannt, daß Menschen, welche unter Streß an Herpes leiden, nach intensiver Sonnenbestrahlung in vielen Fällen ebenfalls Herpesbläschen an den Lippen bekommen.

Bekanntlich wird der gefährlichste Teil der Sonnenstrahlen von den ultravioletten Strahlen mit einer Wellenlänge von weniger als 400 nm gebildet. Bekannt ist auch, daß durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt.

Es ist somit wünschenswert, sowohl medizinische als auch kosmetische Formulierungen zur Verfügung zu stellen, welche die Haut einerseits gegen den schädlichen Einfluß der UV-Strahlung schützen andererseits aber auch eine antivirale Wirkung aufweisen und somit bei vorliegender Herpesinfektion den Ausbruch eines Herpesschubes, der durch Sonnenbestrahlung ausgelöst werden kann, zu verhindern vermögen. Aufgrund eines veränderten Verbraucherverhaltens erscheint es wünschenswert, dieses Problem möglichst durch Verwendung umweltverträgliche Komponenten zu lösen. Insbesondere ist es wünschenswert zu diesem Zweck natürlich vorkommende Substanzen zu verwenden.

Somit ist es Aufgabe der vorliegenden Erfindung kosmetische und/oder medizinische Präparate zur Verfügung zu stellen, die mindestens einen natürlich vorkommenden Lichtschutzfilter enthalten, welche UV-Strahlen in einem Wellenlängenbereich von 280 bis 400 nm absorbieren können, d.h. sowohl UVB-Strahlen mit einer Wellenlänge zwischen 280 und 320 nm, die bei der Bildung eines Sonnenerythems eine entscheidende Rolle spielen, wie auch UVA-Strahlen mit einer Wellenlänge zwischen 320 und 400 nm, welche die Haut bräunen, aber auch altern lassen, die Auslösung einer erythematösen Reaktion begünstigen oder diese Reaktion bei bestimmten Menschen vergrößern oder sogar phototoxische oder photoallergische Reaktionen, oder Herpesschübe auslösen können. Es ist somit auch Aufgabe der vorliegenden Erfindung, Formulierungen zur Verfügung zu stellen, welche eine antivirale Wirkung gegen Herpesviren aufweisen, so daß sie sowohl zur Prophylaxe gegen die Bildung von Herpeserkrankungen der Haut als auch zur Behandlung von entsprechenden Erkrankungen eingesetzt werden können.

Die heute üblichen Sonnenschutzfilter in der Kosmetik werden in UVA- bzw. UVB-Filter unterteilt. Für beide UV-Bereiche gibt es viele aus der Fachliteratur bekannte und bewährte Substanzen, beispielsweise seien hier nur Substanzen wie Phenylbenzimidazol-5-sulfonsäure (Eusolex® 232), Benzophenon-Derivate (Eusolex® 4360), Benzoyl- oder Dibenzoylmethanderivate (Eusolex® 9020 oder Parsol® 1789, Eusolex 8020,), Triazon-Derivate (Uvinul T150®), Salicylat-Derivate (Eusolex HMS®, Eusolex OS®), der Benzylidencampher-Derivate (Eusolex® 6300), Octocyrelen (Eusolex OCR®), anorganische Lichtschutzfilter aus der Gruppe Titandioxid und Zinkoxid aufgeführt.

Die Abstracts von JP 05 27 1088 und JP 05 27 1090 beschreiben Zusammensetzungen mit antiviraler Wirkung, die Glycoflavonoide enthalten. Als Glycoflavonoide werden z.B. Quercetin und Isoquercetin genannt.

Die Lösung der vorliegenden Aufgabe erfolgt durch kosmetische oder medizinische Formulierungen, enthaltend 0,1 Gew.% Isoquercetin.

Solche Formulierungen weisen eine antiviraler Wirkung auf, insbesondere gegen Herpesviren. Gleichzeitig erfolgt die Lösung der Aufgabe auch durch kosmetische oder medizinische Formulierungen, enthaltend 0.1 Gew.% Isoquercetin als UV-Filter mit antiviraler Wirkung.

Gegenstand der Erfindung sind kosmetische oder medizinische Formulierungen enthaltend Isoquercetin in Form einer Creme, Milch, Lotion, eines Öls, eines Stifts, eines Sprays zur Applikation auf der Haut, in Form eines Mund-, Nasen- oder Inhalationssprays, oder in Form von Tabletten, Dragees, Kapseln, Sirup, Saft oder Tropfen.

Erfindungsgemäße Formulierungen können Isoquercetin in Kombination mit einem oder mehreren UVA-Filtern und/ oder einem oder mehreren UVB-Filtern enthalten.

In solchen Formulierungen können UVA-Filter, ausgewählt aus der Gruppe der Benzophenone, Benzoyl- und Dibenzoylmethan-Derivate enthalten sein. Zu den UVA-Filtern zählen auch solche, die im Handel unter den Namen Eusolex® 4360, Eusolex® 8020 oder Eusolex® 9020 erhältlich sind. Erfindungsgemäß enthaltene UVB-Filter können solche sein ausgewählt aus der Gruppe der Zimtsäureester wie Isoamylmethoxycinnamat oder Octylmethoxycinnamat, der Triazine (z. B. Uvinul® T150), Benzylidencampher (Eusolex® 6900), PABA (Eusolex 6007), Octocrylen (Eusolex® OCR), der Salicylate (OS, HMS) und anorganische Lichtschutzfilter wie ZnO und TiO₂. Diese Lichtschutzfilter können einzeln oder in Kombinationen von verschiedenen Lichtschutzfiltern enthalten sein.

Gegenstand der Erfindung sind auch solche Formulierungen, worin die antivirale Wirkung von Isoquercetin durch die Kombination mit Stoffen aus der Gruppe 5-Ethyl-deoxyuridin, Quercetin, Galangin, Kaempferol, Propolis, Chrysin, Apigenin, Luteolin, Myricetin, Acacetin, Vitamine einschließlich der Carotine und Ascorbinsäure oder mit natürlichen Lichtschutzfiltern, wie z. B. Rutin unter Ausnutzung eines synergistischen Effekts verstärkt wird.

Erfindungsgemäße Formulierungen, welche Gegenstand der Erfindung sind, sind dadurch gekennzeichnet, daß darin Isoquercetin in einer Menge von 0.1 Gew.-% enthalten ist.

Vorzugsweise enthalten diese Formulierungen, welche auf die Haut aufzutragen sind, einen Gehalt an Lichtschutzfiltern in der kosmetischen Zubereitung von 0.01 bis 40 Gew.-%.

Entsprechende Formulierungen weisen einen Gehalt an Isoquercetin von 0,1 bis 90 Gew.-% auf bezogen auf die Gesamtmenge in der Formulierung enthaltener Lichtschutzfilter.

Es wurde gefunden, daß die natürlich vorkommende Substanz Isoquercetin als UV-Filter wirksam ist und eine antivirale Wirkung gegen Herpes aufweist. Isoquercetin , das auch unter den Namen Isotrifoliin oder Trifoliin bekannt ist kommt beispielsweise in verschiedenen Pflanzen vor, wie z. B. Grosypium herbaceum, Malvaceae, Ribes nigrum (Schwarze Johannisbeere), Aesculuc hippocastanum L. (Roßkastanie, besonders in den Blüten) , Tropaeolum majus L., Arnica montana L., Corchorus olitorius L., Taxillus levinei, Clematis stans, Hypericum brasiliense und in den Schoten des Dimorphandra-Busches. Isoquercetin gehört mit Rutin und Quercitrin zu dem selben Aglycon: Quercetin. Im Intestinaltract entsteht durch Hydrolyse von Rutin, gegebenenfalls über Isoquercetin als Zwischenprodukt, Quercetin. Pharmakologische Untersuchungen (Chanh et al.; Prostaglandins Leukot. Med. **22**(3), 295 - 300 (1986); Kaul et al., J. Med. Virol. **15** (1), 71 - 79 (1985); Amoros et al.; J. Nat. Prod. **55**(12, 1732 - 1740 (1992); Abou-Karam J. Nat. Prod. **55**(10), 1525 - 1527 (1992)) zeigten jedoch, daß diese Verbindungen, obwohl sie zu dem selben Aglycon gehören, unterschiedliche Wirkungen aufweisen können. Während Quercetin offensichtlich so gut wie keine, bzw nur in sehr hohen Dosen, antivirale Wirkungen aufweist, kann diese für Isoquercetin eindeutig nachgewiesen werden. So wurde gefunden, daß die Infektionsrate durch Herpes simplex Viren vom Typ I nicht durch Quercitrin beeinflußt wird, auch wenn die untersuchten Gewebekulturen in Gegenwart von Quercitrin kultiviert waren.

Isoquercetin ist in reiner Form eine gelbe kristalline Substanz mit einem Schmelzpunkt von 225 bis 227 °C.
Untersuchungen haben ergeben, daß es eine maximale UV-Lichtabsorbtion bei 257 und 359 nm aufweist und daher besonders im UVB-Wellenlängenbereich als UV-Filter wirksam ist, obwohl auch im UVA-Wellenlängenbereich eine Filterwirkung nachzuweisen ist.

Diese in Malvenblüten, welche zur Teezubereitung verwendet werden können, und in Heilpflanzen wie z. b. Arnika oder Roßkastanie vorkommende Verbindung ist daher hervorragend geeignet, in kosmetischen Formulierungen zum Schutz gegen den schädlichen Einfluß von UV-Strahlung als UV-Filter eingearbeitet zu werden.

Durch die nachgewiesene virushemmende Wirkung gegen Herpes simplex Viren wird damit vorteilhafterweise gleichzeitig ein Schutz gegen das Entstehen von Herpesbläschen erzielt, welche bei bestehender Infektion und Sonnenbestrahlung vermehrt auftreten.

Isoquercetin bietet ferner den großen Vorteil, gut hautverträglich zu sein und so gut wie keine toxischen oder allergischen Reaktionen hervorzurufen.

Isoquercetin kann in Konzentrationen bis etwa 40 % in kosmetische Formulierungen eingearbeitet. Es handelt sich dabei, wie schon oben gesagt, um eine kristalline Verbindung, die in üblichen kosmetischen Formulierungen, gegebenenfalls unter Verwendung eines Lösungsvermittlers, gut löslich bzw. gut mischbar ist.

Neben Isoquercetin können zur Verbesserung des UV-Schutzes den Formulierungen weitere natürliche UV-Filter zugefügt werden, wie z. B. die zum gleichen Aglycon zählenden Verbindungen Quercetin oder Rutin, welche als natürlich vorkommende UV-Filter bekannt sind.

Die erfindungsgemäßen Formulierungen können auch zur vorbeugenden Behandlung von Entzündungen und Allergien der Haut, zur Verhütung bestimmter Krebsarten und auch gegebenenfalls bei Zugabe geeigneter Wirkstoffe zur Insektenabwehr verwendet werden.

Unter den oben genannten Bedingungen läßt sich Isoquercetin gleichmäßig in den herkömmlichen kosmetischen Trägern verteilen und kann bei geeigneter Formulierung insbesondere in Fettträgern einen kontinuierlichen Film bilden; sie können auf diese Weise auf die Haut aufgetragen werden, um einen wirksamen Schutzfilm zu bilden.

Formulierungen, welche Isoquercetin enthalten, können gegebenenfalls zur Erhöhung der Fotostabilität von weiteren enthaltenen Lichtschutzfiltern, wie in EP 0 717 982 beschrieben, durch Zugabe einer effektiven Menge einer Amidverbindung verändert werden. Dieses können auch Amidverbindungen sein, wie z. B. 3-(N-Butylacetamino)-propionsäureethylester.

Gegenstand der Erfindung ist somit die Verwendung von Isoquercetin in kosmetischen Formulierungen.

Gegenstand der Erfindung sind auch kosmetische Formulierungen, enthaltend mindestens einen natürlich vorkommenden Lichtschutzfilter wie z. B. Rutin.

In einer bevorzugten Ausführungsform werden neben Isoquercetin Lichtschutzfilter aus der Gruppe der Zimtsäureester wie p-Methoxyzimtsäureoctylester (Eusolex® 2292, Merck KGaA), Isoamylp-methoxyzimtsäureester, Octylmethoxyzimtsäureester und/oder Diethanolamin-p-methoxy-zimtsäureester eingesetzt. Insbesondere bevorzugt wird Eusolex® 2292 verwendet.

Isoquercetin enthaltenden formulierungen können, wie oben schon gesagt, auch Lichtschutzfilter aus der Gruppe der Benzophenone, Benzoyl- und Dibenzoylmethan-Derivate zugesetzt sein oder UVA-Filter, welche beispielsweise im Handel unter den Namen Eusolex® 4360, Eusolex® 8020 oder Eusolex® 9020 erhältlich sind. Sowohl UVA- als auch UVB-Filter können einzeln oder in Kombinationen in erfindungsgemäßen Formulierungen enthalten sein.

Der Gehalt an Lichtschutzfilter in den erfindungsgemäßen kosmetischen Formulierungen beträgt vorzugsweise 0.01 bis 40 Gew.-%, ganz besonders bevorzugt ist ein Gehalt von 1 bis 20 Gew.-%. Eine weitere ganz besonders bevorzugte Ausführungsform enthält 3 bis 10 Gew.-%.

Der Gehalt an Isoquercetin beträgt vorzugsweise 0,1 bis 90 Gew.-%, insbesondere bevorzugt 1 bis 30 Gew.-% bezogen auf die Gesamtmenge an enthaltenem Lichtschutzfilter.

Die natürlich vorkommenden Lichtschutzfilter, ausgewählt aus der Gruppe Isoquercetin, Rutin, Quercetin, Quercitrin, Catechin, Hesperitin können in den erfindungsgemäßen kosmetischen Formulierungen alleine oder in Kombination mit einem oder mehreren UV-Filtern anderer Substanzklassen vorliegen, die jeweils in einer Menge von 0.01 bis 40 Gew.-%, vorzugsweise von 0,1 bis 30 Gew.-% enthalten sein können. Weiterhin können darin Isorhamnetin, Kaempferol, deren Glykoside, Eriodictyol oder oben bereits genannte Stoffe in entsprechenden Mengen enthalten sein und gegebenenfalls die Wirkung des Isoquercetins durch synergistische Effekte verstärken..

Da das Eusolex® 2292, wie schon erwähnt, ein sehr gutes Lösungsmittel für andere UV-Filter darstellt, kann es kombiniert mit anderen UV-Filtern eingesetzt werden, wodurch in hergestellten Formulierungen eine erhöhte Gesamtkonzentration an enthaltenem Lichtschutzfilter erzielt werden kann.

Die Lichtschutzfilter, ausgewählt aus der Gruppe Isoquercetin, Rutin und Quercetin, Quercitrin, Catechin, Hesperitin können als bekannte Naturstoffe im Handel bezogen werden.

Formulierungen, die unter Verwendung insbesondere von Isoquercetin erhalten werden, besitzen als Sonnenschutzmittel neben ihrer antiviralen Wirkung so hervorragende Eigenschaften wie eine sehr gute Haut- und Schleimhautverträglichkeit ohne toxische, allergisierende oder sensibilisierende Eigenschaften zeigen.

Isoquercetin weist als Substanz eine hohe chemische Stabilität, d.h. keine Hydrolysierbarkeit, hohe Thermostabilität und hohe Schweißfestigkeit auf.

Weiterhin ist diese Substanz mit den gängigen kosmetischen und pharmazeutischen Formulierungsgrundlagen gut verträglich und mischbar.

Aufgrund seines Löslichkeitsprofils mischt sich die Substanz gegebenenfalls unter Verwendung von Lösungsvermittlern gut mit Ölen, Fetten und Emulsionen und ist aufgrund ihres Eigenschaftsprofils in Kombination mit anderen bekannten Lichtschutzfiltern einsetzbar. Es ist besonders gut löslich in polaren Lösungsmitteln wie z. B. Ethanol oder 1,2-Propylenglykol. In Glycerin ist es löslich und bildet eine leicht trübe Lösung. Dem Fachmann ist es aufgrund des Eigenschaftsprofils von Isoquercetin leicht möglich je nach gewünschtem Herstellungsprodukt geeignete Lösungsmittel und Additive auszuwählen. Besonders gut lassen sich mit Isoquercetin Emulsionen, Gele oder Emulsionspflegestifte herstellen. Aber auch alle anderen kosmetischen Formulierungen, wie Salben, Cremes, Lotionen, Öle, Stifte und Sprays, sowie pharmazeutische Formulierungen in fester oder flüssiger Form lassen unter Zuhilfenahme geeigneter Zusätze herstellen.

Es ist auch eine Kombination mit 3-(N-Butylacetamino)-propionsäureethylester und Eusolex® 2292 geeignet, um weitere, sonst nur in geringen Konzentrationen lösliche UV-Filter, in die kosmetischen Zubereitungen einzubringen, was mit einer erhöhten Fotostabilität der Formulierungen verbunden ist.

Wird die erfindungsgemäße kosmetische Formulierung zum Schutz menschlicher Epidermis gegen UV-Strahlen verwendet, liegt sie in verschiedenen, für diesen Typ üblicherweise verwendeten Formen vor. So kann sie insbesondere in Form öliger, ölig-wäßriger, wäßrig-alkoholischer oder ölig-alkoholischer Lotionen, Emulsionen, wie als Creme oder als Milch (W/O oder O/W), in Form ölig-alkoholischer, ölig-wäßriger oder wäßrig-alkoholischer Gele oder als feste Stifte oder Puder vorliegen oder als Spray oder Aerosol konfektioniert sein.

Die erfindungsgemäße Formulierung kann weitere kosmetische Adjuvanzien enthalten, welche in dieser Art von Zubereitungen üblicherweise verwendet werden, wie z.B. Verdickungsmittel, weichmachende Mittel, Befeuchtungsmittel, grenzflächenaktive Mittel, Konservierungsmittel, Mittel gegen Schaumbildung, Parfüms, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugt Ausführungsform der Erfindung ist eine Emulsion, welche als Schutzcreme oder -milch vorliegt und außer einem oder mehreren Lichtschutzfiltern und dem Isoquercetin Fettalkohole, Fettsäureester, insbesondere Triglyceride von Fettsäuren, Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Ba-sis von natürlichen oder synthetischen Ölen und Wachsen, Lanolin, Fettsäureestem, insbesondere Triglyceriden von Fettsäuren, oder ölig-alkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestern, wie Triglyceriden von Fettsäuren, dar.

Die erfindungsgemäße kosmetische Zubereitung kann auch als alkoholisches Gel vorliegen, welches einen oder mehrere Niedrigalkohole oder -polyole, wie Ethanol, Propylenglycol oder Glycerin, und ein Verdickungsmittel, wie Kieselerde umfaßt. Die ölig-alkoholischen Gele enthalten außerdem natürliches oder synthetisches Öl oder Wachs.

Eine ganz besonders bevorzugte Ausführungsform besteht in festen Stiften, wie z. b. in Form von Fettstiften für die Lippen oder Sunblokkern. Diese bestehen aus natürlichen oder synthetischen Wachsen und Ölen, Fettalkoholen, Fettsäuren, Fettsäureestem, Lanolin und anderen Fettkörpem, in die die UV-Filter und das Isoquercetin nach dem Fachmann bekannten Methoden eingearbeitet sind.

Ist eine Zubereitung als Aerosol konfektioniert, verwendet man in der Regel die üblichen Treibmittel, wie Alkane, Fluoralkane und Chlorfluoralkane, vorzugsweise CO₂ oder Luft.

Ist eine Formulierung als Spray konfektioniert, verwendet man in der Regel wäßrig-alkoholische Lösungen.

Wie oben schon erwähnt, sind medizinische Formulierungen zur Prävention und Behandlung von Herpesinfektionen oder anderer viraler Infektionen, insbesondere der Haut, ebenfalls Gegenstand der vorliegenden Erfindung.

Isoquercetin und seine physiologisch unbedenklichen Salze können daher zur Herstellung pharmazeutischer Präparate verwendet werden, indem man sie zusammen mit mindestens einem Träger- oder Hilfsstoff und, falls erwünscht, mit einem oder mehreren weiteren Wirkstoffen in die geeignete Dosierungsform bringt. Insbesondere kann Isoquercetin zur Ausnutzung eines synergistischen Effekts hierbei allein oder in Kombination mit weiteren Wirkstoffen eingesetzt werden.

Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z. B. orale) oder parenterale Applikation oder für die Applikation in Form eines Inhalationssprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlenhydrate wie Lactose oder Stärke, Magnesiumstearat, Talk oder Cellulose.

Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen.

Zur parenteralen Applikation dienen Lösungen, vorzugsweise ölige oder wäßrige Lösungen, ferner Suspensionen, Emulsionen oder Implantate. Für die Applikation als Nasen-, Mund- oder Inhalationsspray können Sprays verwendet werden, die den Wirkstoff entweder gelöst oder suspendiert enthalten. Insbesondere in einem Inhalationsspray kann die Verbindung in einem Treibgasgemisch verabreicht werden. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabfolgt werden.

Der erfindungsgemäß beanspruchte Wirkstoff kann auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z. B. ein oder mehrere Vitamine, Diuretika, Antiphlogistika.

Gemäß der Erfindung wird Isoquercetin in der Regel in Analogie zu anderen bekannten, im Handel erhältlichen Präparaten, insbesondere aber in Analogie zu den in der Patentschrift US-PS- 4 880 804 beschriebenen Verbindungen verabreicht, vorzugsweise in Dosierungen zwischen etwa 1 mg und 1 g, insbesondere zwischen 25 und 500 mg pro Dosierungseinheit. Die tägliche Dosierung liegt vorzugsweise zwischen etwa 0.1 und 50 mg/kg, insbesondere 1 und 10 mg/kg Körpergewicht. Die spezielle Dosis für jeden einzelnen Patienten hängt jedoch von den verschiedensten Faktoren ab, beispielsweise von der Wirksamkeit der eingesetzten speziellen Wirkstoffkombination, vom Alter, Körpergewicht, allgemeinem Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt.

Die topische Applikation ist bevorzugt.

Die erfindungsgemäßen kosmetischen Zubereitungen können mit Hilfe von Techniken hergestellt werden, die dem Fachmann wohl bekannt sind.

Auch ohne weitere Ausführungen wird davon ausgegangen, daß ein Fachmann die obige Beschreibung in weitesten Umfang nutzen kann. Die bevorzugten Ausführungsformen sind deswegen lediglich als beschreibende, keineswegs als in irgendeine Weise limitierende Offenbarung aufzufassen.

Die vollständige Offenbarung aller vor- und nachstehend aufgeführten Anmeldungen, Patente und Veröffentlichungen ist durch Bezugnah-me in diese Anmeldung eingeführt.

Die folgenden Beispiele sollen die Erfindung näher erläutern.

### Beispiele

### Beispiel 1

Lippenbalsam mit Isoquercetin

| | | Gew.-% |
|---|---|---|
| A | | |
| Isoquercetin | (1) | 0.1 |
| Cremophor A 25 | (2) Ceteareth-25 | 20.0 |
| Cetiol HE | (3) PEG-7 Glyceryl Cocoate | 22.0 |

| B | | |
|---|---|---|
| Glycerin | Glycerin | 5.0 |
| Konservierungsmittel | | q.s. |
| Demin. Wasser | Aqua | ad 100 |

### Herstellung

Phase A und B werden auf eine Temperatur von 80 °C erwärmt. B wird unter Rühren zu A gegeben und auf Raumtemperatur abgekühlt.

Es wird ein farbloses Gel erhalten.

Lieferanten:
(1) Merck KGaA, Darmstadt
(2) BASF
(3) Henkel KGaA, Düsseldorf

## Patentansprüche

1. Kosmetische oder medizinische Formulierungen, enthaltend 0.1 Gew.% Isoquercetin als UV-Filter mit antiviraler Wirkung gegen Herpesviren.

2. Formulierung gemäß Anspruch 1, in Form einer Salbe, Creme, Milch, Lotion, einer Emulsion, eines Öls, eines Gels, eines Stiftes, eines Sprays zur Applikation auf der Haut.

3. Formulierung gemäß Anspruch 1 in Form eines Mund-, Nasen- oder Inhalationssprays.

4. Formulierung gemäß Anspruch 1 in Form von Tabletten, Dragees, Kapseln, Sirup, Saft oder Tropfen.

5. Formulierung gemäß Anspruch 1, enthaltend Isoquercetin in Kombination mit einem oder mehreren UVA-Filtern und/oder einem oder mehreren UVB-Filtern.

6. Formulierung gemäß Anspruch 5, **dadurch gekennzeichnet, dass** der Gehalt an Lichtschutzfiltern in der kosmetischen Zubereitung 0.01 bis 40 Gew.% beträgt.

7. Formulierung gemäß Anspruch 5 enthaltend UV-Filter, ausgewählt aus der Gruppe Benzophenone, Benzoyl- und Dibenzoylmethan-Derivate, Zimtsäureester wie Isoamylmethoxycinnamat oder Octylmethoxycinnamat, Triazine, Benzylidencampher, PABA, Salicylate, Octocrylen, ZnO und TiO₂.

8. Formulierung gemäß einem oder mehreren der Ansprüche 5 bis 7, **dadurch gekennzeichnet, dass** der Gehalt an Isoquercetin im Bereich von 0.1 bis 90 Gew.% bezogen auf die Gesamtmenge der in der Formulierung enthaltenen Lichtschutzfilter liegt.

9. Formulierung gemäß einem oder mehreren der vorhergehenden Ansprüche, **dadurch gekennzeichnet, dass** die antivirale Wirkung von Isoquercetin durch die Kombination mit Stoffen aus der Gruppe 5-Ethyldeoxyuridin, Quercetin, Galangin, Kaempferol, Propolis, Chrysin, Apigenin, Luteolin, Myricetin, Acacetin, Eriodictyol, Isorhamnetin, sowie dessen Glykosid, Vitamine einschließlich der Carotine und Ascorbinsäure, und/oder mit natürlichen Lichtschutzfiltern aus der Gruppe Quercetin, Quercitrin, Catechin, Hesperitin und Rutin, sowie dessen Glykosid unter Ausnutzung eines synergistischen Effekts verstärkt wird.

## Claims

1. Cosmetic or medical formulation comprising 0.1% by weight of isoquercetin as UV filter, having an antiviral action against herpes viruses.

2. Formulation according to Claim 1, in the form of an ointment, cream, milk, lotion, emulsion, oil, gel, stick, spray for application to the skin.

3. Formulation according to Claim 1 in the form of an oral, nasal or inhalation spray.

4. Formulation according to Claim 1 in the form of tablets, dragées, capsules, syrup, juice or drops.

5. Formulation according to Claim 1, comprising isoquercetin in combination with one or more UVA filters and/or one or more UVB filters.

6. Formulation according to Claim 5, **characterised in that** the content of light-protection filters in the cosmetic preparation is 0.01 to 40% by weight.

7. Formulation according to Claim 5 comprising UV filters selected from the group consisting of benzophenones, benzoyl- and dibenzoylmethane derivatives, cinnamic acid esters, such as isoamyl methoxycinnamate or octyl methoxycinnamate, triazines, benzylidenecamphor, PABA, salicylates, octocrylene, ZnO and TiO₂.

8. Formulation according to one or more of Claims 5 to 7, **characterised in that** the content of isoquercetin is in the range from 0.1 to 90% by weight, based on the total amount of light-protection filters present in the formulation.

9. Formulation according to one or more of the preceding claims, **characterised in that** the antiviral action of isoquercetin is augmented by combination with substances from the group consisting of 5-ethyldeoxyuridine, quercetin, galangin, kaempferol, propolis, chrysine, apigenin, luteolin, myricetin, acacetin, eriodictyol, isorhamnetin, and the glycoside thereof, vitamins, including the carotenes and ascorbic acid, and/or with natural light-protection filters from the group consisting of quercetin, quercitrin, catechol, hesperitin and rutin, and the glycoside thereof, utilising a synergistic effect.

## Revendications

1. Formulation cosmétique ou médicale comprenant 0,1% en poids d'isoquercétine en tant que filtre UV, présentant une action antivirale contre les virus de l'herpès.

2. Formulation selon la revendication 1, sous la forme d'une pommade, d'une crème, d'un lait, d'une lotion, d'une émulsion, d'une huile, d'un gel, d'un bâton, d'un spray pour une application sur la peau.

3. Formulation selon la revendication 1, sous la forme d'un spray oral, nasal ou d'inhalation.

4. Formulation selon la revendication 1, sous la forme de comprimés, de dragées, de gélules, de sirop, de jus ou de gouttes.

5. Formulation selon la revendication 1, comprenant de l'isoquercétine en combinaison avec un ou plusieurs filtres UVA et/ou un ou plusieurs filtres UVB.

6. Formulation selon la revendication 5, **caractérisée en ce que** la teneur en filtres protecteur de la lumière dans la préparation cosmétique est de 0,01 à 40% en poids.

7. Formulation selon la revendication 5, comprenant des filtres UV sélectionnés parmi le groupe constitué par des benzophénones, des dérivés de benzoyl- et de dibenzoylméthane, des esters d'acide cinnamique, tels que méthoxycinnamate d'isoamyle ou méthoxycinnamate d'octyle, des triazines, du benzylidènecamphre, du PABA, des salicylates, de l'octocrylène, du ZnO et du TiO₂.

8. Formulation selon une ou plusieurs des revendications 5 à 7, **caractérisée en ce que** la teneur en isoquercétine est dans la plage qui va de 0,1 à 90% en poids, sur la base de la quantité totale de filtres de protection vis-à-vis de la lumière présente dans la formulation.

9. Formulation selon une ou plusieurs des revendications précédentes, **caractérisée en ce que** l'action antivirale de l'isoquercétine est augmentée au moyen d'une combinaison avec des substances provenant du groupe constitué par 5-éthyldéoxyuridine, quercétine, galangine, kaempferol, propolis, chrysine, apigénine, lutéoline, myricétine, acacétine, ériodictyol, isorhamnétine, et leurs glycosides, des vitamines, incluant les carotènes et l'acide ascorbique, et/ou avec des filtres de protection vis-à-vis de la lumière naturelle pris parmi le groupe constitué par quercétine, quercitrine, catéchol, hespéritine et rutine, et leurs glycosides, en utilisant un effet synergique.
